# EUROPÄISCHE PATENTSCHRIFT

(11) **EP 2 953 919 B1**
(45) Veröffentlichungstag und Bekanntmachung des Hinweises auf die Patenterteilung: **19.10.2016**
(21) Anmeldenummer: 14702783.3
(22) Anmeldetag: 28.01.2014
(51) Int. Cl.: C07C 45/35, C07C 45/78, C07C 45/82, C07C 47/22

(54) **VERFAHREN ZUR ABTRENNUNG VON ACROLEIN AUS DEM PROZESSGAS EINER HETEROGEN KATALYSIERTEN OXIDATION VON PROPEN**
PROCESS FOR THE SEPARATION OF ACROLEIN FROM THE PROCESS GAS FROM THE HETEROGENEOUSLY CATALYZED OXIDATION OF PROPENE
PROCÉDÉ DESTINÉ À SÉPARER L'ACROLÉINE DEPUIS LE GAZ DE PROCESSUS D'UNE OXYDATION CATALYSÉE HÉTÉROGÈNE DE PROPÈNE

(30) Priorität: 06.02.2013 EP 13154227
(43) Veröffentlichungstag der Anmeldung: 16.12.2015
(73) Patentinhaber: Evonik Degussa GmbH, 45128 Essen (DE)
(72) Erfinder: BÖCK, Wolfgang, 63505 Langenselbold (DE); TAUGNER, Wolfgang, 63571 Gelnhausen (DE); GRUBER, Udo, 64289 Darmstadt (DE)
(86) Internationale Anmeldenummer: PCT/EP2014/051573
(87) Internationale Veröffentlichungsnummer: WO 2014/122044

(56) Entgegenhaltungen:
- WO-A2-01/96271
- DE-A1- 2 263 496

## Beschreibung

Die heterogen katalysierte Oxidation von Propen mit Luftsauerstoff nach dem gegenwärtigen Stand der Technik wird in den neuesten Auflagen von Ullmann's Enzyclopedia of Industrial Chemistry, D. Arntz et al., online, Edition 15.04.2007, Band 1, Wiley-VCH-Verlag und Kirk-Othmer Enzyclopedia of Chemical Technology, W. G. Etzhorn, online, Edition 18.12.2009, Band 1, John Wiley and Sons Inc. umfassend beschrieben.

Die Oxidation des Propen erfolgt dabei an in Rohrbündelrekatoren eingebrachten Katalysatoren bei erhöhten Temperaturen (300 - 400 °C) und leicht erhöhtem Druck. Dem Feedgas werden neben Propen und Luft oft auch Dampf und andere Inertgase zugemischt, um mit der Feedgaszusammensetzung außerhalb der Zündgrenzen zu bleiben. Die Reaktionswärme wird meist durch eine flüssige Salzschmelze, die durch den Mantelraum des Rohrbündelreaktors zirkuliert, abgeführt. Als geeignete Katalysatoren wird eine Vielzahl unterschiedlicher Mischoxidsysteme angegeben, die als Basis Molybdän, Wismut, Eisen, Kobalt, Nickel und weitere Promotoren enthalten, wie beispielsweise angegeben in PCT-Anmeldung WO 2008/046843 oder WO 2008/104432. Aus vielen Patentschriften sind verschiedenste weitere derartige Mischoxidsysteme bekannt, die die erzielbare Acroleinausbeute erhöhen. Es werden dabei sowohl Vollkatalysatoren (z. B. als Tabletten oder Hohlzylinder) als auch Trägerkatalysatoren (z. B. als Schalenkatalysatoren) eingesetzt. Allen Reaktionssystemen gemeinsam ist, dass bei der Oxidationsreaktion neben dem Zielprodukt Acrolein auch diverse weitere Nebenprodukte gebildet werden (u.a. Acrylsäure, Essigsäure, Formaldehyd, Acetaldehyd, Allylalkohol, CO, CO2).

Um das Acrolein als Einsatzstoff für weitere Synthesereaktionen verwenden zu können, muss es aus diesem Reaktionsgemisch abgetrennt und aufgereinigt werden. Die prinzipielle Vorgehensweise ist dazu ebenfalls in obigen Literaturstellen nachzulesen. Zunächst ist das Reaktionsgas abzukühlen und dann aus dem Reaktionsgas die hochsiedenden Anteile und Wasser abzutrennen. Das hochsiederhaltige anfallende Abwasser wird üblicherweise thermisch entsorgt. Es können aber auch andere Entsorgungswege eingeschlagen werden. Aus dem so vorgereinigten Prozessgasstrom wird dann das Acrolein ausgewaschen (absorbiert), da eine Auskondensation aufgrund der Verdünnung und der niedrigen Siedetemperatur vollkommen unwirtschaftlich wäre. Aus der erhaltenen Sorbatlösung werden das Acrolein und die noch vorhandenen Leichtsieder abdestilliert. Die von Acrolein befreite Sorptionsflüssigkeit kann dann erneut zur Absorption von Acrolein eingesetzt werden. Als Sorptionsflüssigkeit wird üblicherweise Wasser eingesetzt oder ein Gemisch aus Wasser und einem organischen Lösungsmittel.

Falls nötig, kann das Acrolein im Weiteren zur Abtrennung der Leichtsieder noch weiter destilliert werden. Aus den genannten und vielen weiteren Literaturstellen ist bekannt, dass Acrolein, bzw. Acrylsäure, Formaldehyd oder Allylalkohol leicht polymerisieren. Daher wird im Aufarbeitungsteil bzw. dem Acrolein selbst Stabilisator zugesetzt, um die Polymerisationsneigung zu verringern. Als geeignet hat sich dafür z.B. Hydrochinon erwiesen, es werden jedoch auch eine Vielzahl anderer Stabilisatorsysteme beschrieben. Auch durch den Zusatz von Stabilisator kann eine Polymerisation im Aufarbeitungsteil nicht 100%ig verhindert werden. Insbesondere durch die thermische Belastung bei der Abtrennung des Acrolein aus der Sorptionsflüssigkeit werden immer in kleinem Umfang Oligomere gebildet, die sich über die Zeit in der Sorptionsflüssigkeit anreichern und zur Bildung von Polymeren führen, die dann wiederum eine Verschmutzung der Produktionseinrichtungen mit der Notwendigkeit einer Reinigung verursachen.

DE 2263496 beschreibt ein Verfahren zur Herstellung von Acrylsäure und Acrolein, bei dem das aus einer katalytischen Gasphasenoxidation von Propen kommende Acrolein- und Acrylsäure-haltige Gasgemisch zur extraktiven Abtrennung von Acylsäure durch eine Waschflüssigkeit, die eine Mischung aus Wasser und organischer Flüssigkeit ist, geleitet wird. Die dabei entstehende hochsiedende, Acrylsäure-haltige wässrig-/organische Lösung wird als Sumpfphase abgetrennt. Als hochsiedendes organisches Extraktionsmittel wird insbesondere 2-Ethylhexanol verwendet. Die Acrylsäure wird in mehreren Aufarbeitungsschritten destillativ von der wässrig-/organischen Lösung abgetrennt. Dazu wird zunächst Wasser abdestilliert und die Acrylsäure aus der verbliebenen organischen Flüssigkeit abdestilliert und dann die organische Flüssigkeit als Waschflüssigkeit wiederverwendet. Nachteil des Verfahrens ist, dass relativ große Mengen Lösungsmittel verwendet und im Kreis gefahren werden müssen, die in einem aufwendigen Prozess wiedergewonnen werden müssen, das bedeutet einen nicht unerheblichen Energieaufwand sowie eine weitere Quelle zusätzlicher Verunreinigungen, welche insbesondere durch die Reaktion des Lösungsmittels mit Acrylsäure entstehen.

Neben der möglichst effizienten Abtrennung von Nebenprodukten aus dem Acroleinprozess ist es ferner notwendig, eine Ablagerung von sich bildenden Polymernebenprodukten in den Apparaten (zum Beispiel Kolonnen, Kolonneneinbauten, Wärmetauschern) und Rohrleitungen zu verhindern, die schnell zu einer Verschlechterung der Wärmedurchgänge oder des Anlagendurchsatzes führen würden. Dies kann zwar prinzipiell auch durch Verwendung von Wasser/LösungsmittelGemischen als Waschlösung insbesondere in der Quenchstufe geschehen, wie es in einem Verfahren gemäß DE 2263496 stattfindet. Nachteil dabei ist aber vor allem der gesonderte Aufwand für dessen Reinigung und Recyclierung.

WO 2010044990 A1 beschreibt dagegen ein Verfahren zur extraktiven Abtrennung von Acrylsäure aus dem Abwasser eines Acroleinprozesses mit Hilfe von verflüssigten C3-Gasen Propylen und/ oder Propan als Extraktionsmittel mit dem Ziel Acrylsäure zurückzugewinnen. Dieses Verfahren ermöglicht die leichte destillative Abtrennung des Lösungsmittels von der Acrylsäure-haltigen Extraktionslösung, was einen Vorteil gegenüber höhersiedenden Extraktionsmitteln darstellt.

WO2001096271 offenbart schließlich die Herstellung von Acrolein beginnend mit einer Absorption von Propylen/Propan in ein organisches Lösungsmittel (C₈₋₂₀-Alkan oder Alken), bei dem Propylen/Propan anschließend z. B. destillativ (Anspruch 4) vom Absorptionsmittel abgetrennt wird (Ansprüche 1,3). Mitgerissenes Absorptionsmittel wird danach mit Wasser aus dem Propylen/Propan-Strom ausgewaschen. Das erhaltene Gemisch aus Wasser/organische Lösungsmittel wird durch Phasentrennung aufbereitet, damit die beiden Phasen getrennt in den Prozess wiederverwendet werden (Seite 59, Zeile 1 - Seite 60, Zeile 8).

Für die Wirtschaftlichkeit eines Produktionsprozesses ist es allgemein von besonderer Bedeutung, dass die Betriebsdauer zwischen notwendigen Reinigungsstillständen möglichst lange sowie der Energieverbrauch im laufenden Betrieb möglichst niedrig ist.

Durch den Einsatz verbesserter katalysatoren wie beispielsweise beschrieben in WO 2008/104432 oder WO 2008/046843 ist es mittlerweile gelungen den Acyrlsäureanteil im Acroleinprozess weiter zu reduzieren, so dass ein Verfahren mit Verwendung von großen Mengen von Lösungsmitteln zur Abtrennung der Acrylsäure gemäß DE 2263496 wirtschaftlich noch weniger attraktiv geworden ist.

Die vorliegende Erfindung hat daher zur Aufgabe, einen Prozess zur Abtrennung von Acrolein aus dem Prozessgas einer heterogen katalysierten Oxidation von Propen bereitzustellen, der unter Zusatz eines organischen Lösungsmittels im Aufarbeitungsteil eine Erhöhung der Betriebszeit der Anlage ermöglicht, dabei jedoch die Nachteile der bekannten Prozesse vermeidet, sowie insbesondere eine Unterdrückung von Schaumbildung in den Kolonnen und eine Durchsatzerhöhung in bestehenden Anlagen erlaubt sowie gleichzeitig eine möglichst einfache und kostengünstige Aufarbeitung und Recyclierung des eingesetzten Lösungsmittels ermöglicht.

### Beschreibung der Erfindung

Es gelingt erfindungsgemäß, die Nachteile des Standes der Technik zu überwinden, durch Bereitstellung eines kontinuierlichen Verfahrens zur Herstellung von Acrolein, bei dem die nachfolgenden Schritte durchgeführt werden
A) Gasphasenoxidation von Propen mit Hilfe von Luft an einem heterogenen Katalysator in Anwesenheit eines Verdünnungsgases in einer Reaktionsstufe unter Erzeugung eines Acrolein-haltigen Gasstromes (Prozessgas)
B) Auffangen des Acrolein-haltigen Gasstromes aus A) in einer Quenchstufe bestehend aus Sumpf-, Mittel- und Kopfteil zur Abtrennung von Nebenprodukten in Gegenwart von Wasser und einer kaum wasserlösliches organisches Lösungmittel enthaltenden organischen Phase
C) Auffangen des Acrolein-haltigen Gasstromes aus der Quenchstufe B) in einer Absorptionsstufe bestehend aus Sumpf-, Mittel- und Kopfteil in Gegenwart von Wasser und einer kaum wasserlösliches organisches Lösungmittel enthaltenden organischen Phase zur Gewinnung einer organische Phase enthaltenden wässrigen Acrolein-Lösung und eines nicht kondensierbaren Gasstromes
C1) zumindest teilweise Rückführung des nicht kondensierbaren Gasstromes aus C) als Verdünnungsgas in die Reaktionsstufe A)
D) Destillative Abtrennung des Acroleins aus der organische Phase enthaltenden wässrigen Acrolein-Lösung aus C) in einer Destillationsstufe,
wobei die verbleibende vom Acrolein befreite, organische Phase enthaltende wässrige Lösung aus der Destillationsstufe D) ausgetragen wird, so dass sich eine organische Phase II bildet, die vom zugehörigen wässrigen Anteil II getrennt wird (Phasentrennung II), die organische Phase II in den Sumpfteil der Quenchstufe B) eingetragen wird, aus welchem die organische Phase II durch Destillation und /oder Strippung zusammen mit einem wässrigen Anteil über den Kopfteil abgetrennt wird, so dass sich eine organische Phase I bildet, welche vom zugehörigen wässrigen Anteil I getrennt wird ( Phasentrennung I) und in den Sumpfteil der Absorptionsstufe C) eingetragen wird.

Unter einem kaum wasserlöslichen organischen Lösungsmittel wird insbesondere ein organisches Lösungsmittel verstanden, dass eine Wasserlöslichkeit vorn ≤ 20g/l, vorzugsweise vorn ≤ 6,0g/l, besonders bevorzugt vorn ≤ 2,0g/l jeweils bei 20°C aufweist.

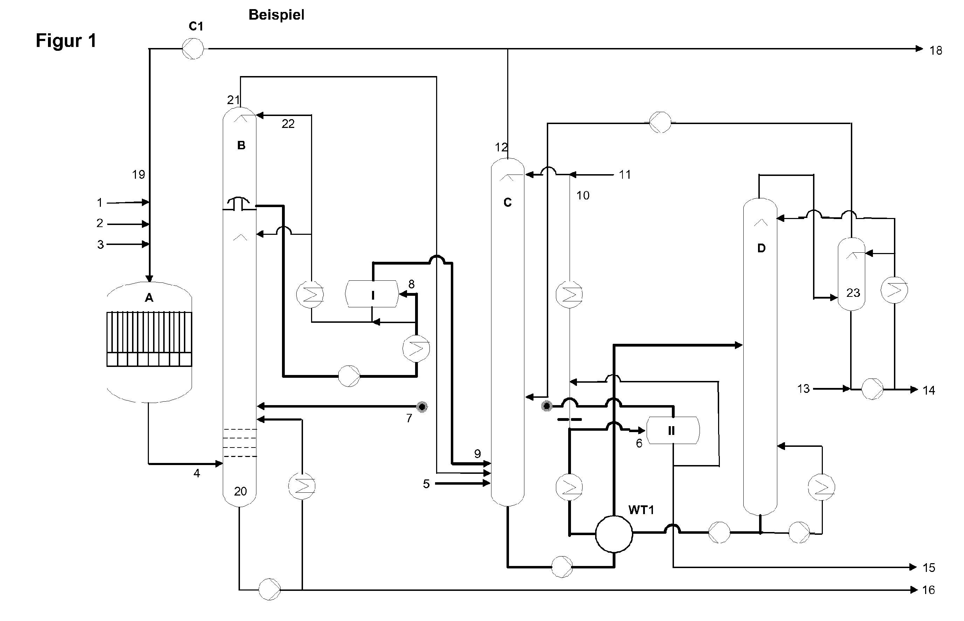

Im Fall der Ausführung der Quenchstufe B) in Form einer Kolonne ist am unteren Ende des Kopfteils vorzugsweise ein Fangboden installiert, über dem sich der Austrag der organisch/wässrigen Phase zum Phasentrenner I befindet (vgl. Figur 1), so dass aus dem so entstandenen Zwischensumpf besonders gut Flüssigkeit ausgetragen werden kann.

In Schritt C1 können auch ergänzend zu oder anstelle des nicht kondensierbaren Gasstromes (nicht kondensierbaren Prozessgases) weitere Inertgase, wie zum Beispiel Stickstoff oder Wasserdampf oder sauerstoffarmes Abgas aus einer Verbrennungsanlage als Verdünnungsgas verwendet werden.

Das erfindungsgemäße Verfahren ermöglicht insbesondere durch den Zusatz eines organischen Lösungsmittels und die sich daraus bildenden organischen Phasen im Aufarbeitungsteil die Abtrennung von potentiell Polymer bildenden Verunreinigungen und damit eine Erhöhung der Betriebszeit der Anlage und dabei gleichzeitig, das eingesetzte Lösungsmittel intern zu rezirkulieren und destillativ zu reinigen, ohne dass es eines zusätzlichen apparativen Aufwands bedarf. Auch wird die Schäumungsneigung im System insbesondere in der Destillationsstufe reduziert. Das Verfahren ermöglicht somit insgesamt eine Erhöhung der Betriebszeit der Anlage und des Durchsatzes an Edukt- bzw. Produktströmen.

Bevorzugt wird dabei ein Verfahren bei dem permanent oder intermittierend kaum wasserlösliches organisches Lösungsmittel zur Ergänzung der organischen Phase an geeigneter Stelle des Verfahrens eingebracht wird. So wird insbesondere das Konstanthalten des Gehalts an organischer Phase und dadurch eine stabile Fahrweise ermöglicht.

Besonders bevorzugt ist es ferner, dass das organische Lösungsmittel in den Sumpfteil der Absorptionsstufe und/oder der Destillationsstufe eingebracht wird. Durch den Eintrag in die Sumpf der Absorptionsstufe wird insbesondere die Hauptfunktion der Absorptionsstufe, die Abtrennung des Acroleins aus dem Quenchgas aus der Stufe B) am wenigsten beeinträchtigt. Das organische Lösungsmittel wird dazu vorzugsweise in den Zufuhrstrom der organischen Phase zur Absorptionsstufe eingemischt oder separat unterhalb dieser Position eingeführt. Im Fall des Lösungsmitteleintrags in den Sumpf der Destillationsstufe wird durch diese Maßnahme auch das Durchschlagen des Lösungsmittels bzw. der organischen Phase in den Kopfteil der Destillationsstufe und damit ins Produkt Acrolein verhindert.

Vorzugsweise eingesetzt wird ein organisches Lösungsmittel, das einen Siedepunkt bei Normaldruck von 150 bis 230 °C, besonders bevorzugt 170 bis 190 °C aufweist. Dadurch ist insbesondere die Austragung des Lösungsmittels vom Sumpfteil in den Kopfteil durch Strippung und Destillation in der Quenchstufe gewährleistet. Als Strippgas dient hier insbesondere das Prozessgas.

Besonders bevorzugt wird als organisches Lösungsmittel ein verzweigter bzw. unverzweigter offenkettiger C6- bis C10-Alkohol, insbesondere 2-Ethylhexanol verwendet, welches auch großtechnisch in hinreichenden Mengen zu Verfügung steht. Da die Alkohole mit im Prozess auftretenden Substanzen reagieren, z.B. mit Acrylsäure unter Bildung von Acrylsäureestern, also im Falle von 2-Ethylhexanol zu 2-Ethylhexylacrylat bzw. mit Essigsäure zu 2-Ethylhexylacetat und mit Formaldehyd zu 2-Ethylhexylformal und 2-Ethylhexyldiformal, werden diese Prozessprodukte zusammen mit dem Lösungsmittel Bestandteil der erfindungsgemäß verwendeten organischen Phasen, welche in diesem Zusammenhang auch als Lösungsmittelmatrix (LM) bezeichnet werden. Dies ist im vorliegenden Prozess sogar von Vorteil, denn weiterhin bevorzugt sollte die Lösungsmittelmatrix auch eine gute Aufnahmefähigkeit für gebildete Oligomere und weitere organische Nebenprodukte aufweisen, was mit der aus den genannten Alkoholen gebildeten organischen Phasen in besonderer Weise der Fall ist.

Weiter bevorzugt ist beim erfindungsgemäßen Verfahren, dass bezogen auf die in die Reaktionsstufe A) eingetragenen Menge an Propen zwischen 0,05 und 1 Gew.%, vorzugsweise zwischen 0,1 und 0,5 Gew.% organisches Lösungsmittel ergänzt wird, vorzugsweise soviel organisches Lösungsmittel ergänzt wird, dass ein gleichbleibender Gehalt an organischer Phase aufrechterhalten wird. Dies kann durch Gehaltsbestimmung der organischen Phasen, insbesondere der organischen Phase im Ausgangsstrom der Phasentrennungen, insbesondere der Phasentrennung II durch bekannte Analysenmethoden überprüft und entsprechend nachjustiert werden. Dadurch wird eine besonders vorteilhafte und gleichmäßige Fahrweise des erfindungsgemäßen Verfahrens und eine Minimierung des Lösungsmittelverbrauchs ermöglicht.

Weiter bevorzugt ist beim erfindungsgemäßen Verfahren auch, dass der Acrolein-haltige Gasstrom aus A) mit einer Temperatur von 200-280°C, insbesondere von 220-250°C in den Sumpfteil des entsprechenden Schritts B) gelangt. Das Prozessgas wird dazu bereits im Nachkühler der Reaktionsstufe A entsprechend abgekühlt, um den Kühlwasserbedarf in der Quenchstufe abzusenken. Höhere Temperaturen würden darüberhinaus die Bildung von Nebenprodukten verstärken. Somit wird eine energetisch und im Hinblick auf maximale Acrolein-Selektivität optimierte Fahrweise der Kombination aus der Reaktionsstufe A) und der nachfolgenden Quenchstufe B) ermöglicht.

Weiter bevorzugt ist beim erfindungsgemäßen Verfahren, dass in der Quenchstufe B die Sumpftemperatur zwischen 60 und 90 °C bei einem Druck im Kopfteil von 1,2 bis 1,8 bar (Absolutdruck) so eingestellt wird, dass keine separate organische Phase im Sumpfteil der Quenchstufe B vorhanden ist. Damit wird der Sumpfaustrag der im Prozess benötigten organischen Phase verhindert und auch gewährleistet, dass die flüchtigen Anteile der organischen Phase erfindungsgemäß in den Kopfteil der Quenchstufe B transportiert werden.

Besonders bevorzugt wird dabei die Sumpftemperatur der Quenchstufe B eingestellt, indem ein Teilstrom der dort kondensierten Flüssigkeit abgeführt wird und nach entsprechendem Abkühlen dem unteren Drittel der Quenchstufe B wieder zugeführt wird (unteres Umpumpen). Die gleichmäßige Beschickung und Temperatureinstellung insbesondere via externe Wärmetauscher wird auf diese Weise möglich.

Ganz besonders bevorzugt wird dabei, dass aus dem abgeführten Teilstrom der kondensierten Flüssigkeit ein Teilstrom aus dem Verfahren ausgeschleust wird. Dadurch werden Acrylsäure und weitere Nebenprodukte, insbesondere die in der organischen Phase II enthaltenen hochsiedenden Nebenprodukte, die nicht mit der organischen Phase II abgestrippt werden und das gebildete Reaktionswasser aus dem Sumpfteil der Quenchstufe und aus dem Verfahren ausgeschleust. Damit wird ein konstantes Flüssigkeitsniveau im Sumpf der Kolonne sowie ein weitgehend konstanter Anteil von Nebenprodukten erreicht.

Ebenso bevorzugt ist beim erfindungsgemäßen Verfahren, dass ein Teilstrom der im Kopfteil der Quenchstufe B vorhandenen Kondensats abgeführt wird, vorzugsweise auf < 20°C gekühlt und dem Kopfteil der Quenchstufe B wieder zugeführt wird, wodurch der aus dem Mitteilteil der Quenchstufe B eintretende Acrolein-haltige Gasstrom gekühlt wird und fortwährend weiteres Kondensat erzeugt wird, welches als Rücklauf in den Mittelteil der Quenchstufe zurückgeführt wird (oberes Umpumpen). Dies ermöglicht vor allem eine gleichmäßige Beschickung und Temperatureinstellung insbesondere via externen Wärmetauscher zur Kondensation von wässriger und organischer Phase bzw. Lösungsmittel.

Ebenso bevorzugt ist beim erfindungsgemäßen Verfahren, dass ein Teilstrom des Kondensats aus dem Kopfteil der Quenchstufe B der Phasentrennung I zugeführt wird. Dadurch ist eine In situ- Abtrennung der organischen Phase I auf sehr einfache und effiziente Weise möglich zur sofortigen Wiederverwendung im Verfahren.

Auch bevorzugt ist beim erfindungsgemäßen Verfahren, dass die Phasentrennung I bei einer Temperatur von 8 bis 60 °C, vorzugsweise von 10 bis 50 °C besonders bevorzugt von 12 bis 40°C durchgeführt wird. Dadurch wird insbesondere eine optimale Abtrennung der organischen Phase mit dem vorhandenen Lösungsmittel bei minimalen Verlusten erreicht.

Erfindungsgemäß weiter bevorzugt ist ein Verfahren bei dem die Phasentrennung I mit einer Verweilzeit von 0,5 bis 20 min, vorzugsweise von 1 bis 10 min durchgeführt wird. Auch diese Bedingungen tragen zu einer optimalen Abtrennung der organischen Phase mit dem vorhandenen Lösungsmittel bei minimalen Verlusten bei.

Erfindungsgemäß bevorzugt ist auch ein Verfahren, bei dem der wässrige Anteil I aus der Phasentrennung I zumindest teilweise dem Rücklauf des erzeugten Kondensats aus der Quenchstufe B zugeführt wird. Dadurch wird der Durchbruch von Acrylsäure in die Absorptionsstufe weitgehend verhindert und die Anreicherung von LM im Mittelteil der Quenchkolonne verringert.

Erfindungsgemäß bevorzugt ist weiter ein Verfahren, bei dem die aus der Destillationsstufe D ausgetragene vom Acrolein befreite, organische Phase enthaltende wässrige Lösung (Sorptionslösung) teilweise direkt auf den Kopfteil der Absorptionsstufe C zurückgeführt wird und der restliche Teil des Phasentrennung II zugeführt wird.

Die Sorptionslösung aus der Destillation wird dabei sofort in situ- und praktisch verlustfrei verwertet sowie die Gesamtmenge der Abfallströme minimiert. Durch diese Fahrweise lässt sich die Verweilzeit in der Phasentrennung II so einstellen, dass eine optimale Abtrennung der organischen Phase möglich wird.

Für die optimale Funktion des erfindungsgemäß betriebenen Phasentrenners II ist dabei neben einer hinreichend langen Verweilzeit die Wahl einer geeigneten Temperatur entscheidend. Bevorzugt ist daher ein Verfahren bei dem die Phasentrennung II bei einer Temperatur von 10 bis 90 °C, vorzugsweise von 15 bis 60 °C besonders bevorzugt von 20 bis 40°C durchgeführt wird. Damit wird die optimale Abtrennung der organischen Phase mit dem vorhandenen Lösungsmittel bei den gegebenen Viskositäten unter minimalen Verlusten sichergestellt.

Erfindungsgemäß bevorzugt wird auch ein Verfahren, bei dem die Phasentrennung II mit einer Verweilzeit von 0,5 bis 20 min, vorzugsweise von 1 bis 10 min durchgeführt wird. Das trägt ebenfalls zu einer optimalen Abtrennung der organischen Phase mit dem vorhandenen Lösungsmittel bei minimalen Verlusten bei, ohne dass die Wirtschaftlichkeit des Verfahrens leidet.

Erfindungsgemäß weiter bevorzugt wird ein Verfahren, bei dem die Menge an organischer Phase II, die in die Quenchstufe B) eingetragen wird 0,1 bis 3 Gew.%, vorzugsweise 0,2 bis 1 Gew.%, bezogen auf die insgesamt in die Reaktionsstufe A) eingetragenen Mengen an Propen beträgt. Dies gewährleistet eine optimale Entfaltung der Wirkung der organischen Phase bei minimaler Belastung durch die zusätzlich eingebrachten Stoffströme.

Erfindungsgemäß bevorzugt wird auch ein Verfahren bei dem der wässrige Anteil II aus der Phasentrennung II teilweise zur Absorptionsstufe C), vorzugsweise zum Kopfteil der Absorptionsstufe, zurückgeführt wird und vorzugsweise ein weiterer Teilstrom des wässrigen Anteils II aus dem Verfahren ausgeschleust wird. Dadurch wird das Prozesswasser sofort und praktisch verlustfrei verwertet sowie die Gesamtmenge der Abfallströme minimiert. Durch die Ausschleusung wird die Gesamtmenge der Flüssigkeit im Verfahren (hold-up) kontrolliert und die Anreicherung von Nebenprodukten in der wässrigen Phase vermieden.

Erfindungsgemäß bevorzugt wird weiter ein Verfahren bei dem der Gehalt an organischer Phase in der Sorptionslösung 0,1 bis 5 Gew.% beträgt, vorzugsweise 0,2 bis 3 Gew.%. Der Gehalt an Lösungsmittel-haltiger organischer Phase ist damit so hoch, dass sich eine abtrennbare organische Phase II ausbildet.

Erfindungsgemäß wird auch bevorzugt ein Verfahren bei dem die Sorptionslösung mit einer Temperatur von 5 - 18°C, vorzugsweise 6 - 12°C und bei einem Druck am Kopfteil der Absorptionsstufe C) zwischen 1,1 und 1,7 bar in die Absorptionsstufe C) eingebracht wird. Dies gewährleistet eine weitgehende Absorption von Acrolein aus dem Quenchgasstrom.

Beim erfindungsgemäßen Verfahren sind die Quench- , Absorptions- und/oder Destillationsstufe jeweils vorzugsweise in Form einer Kolonne ausgeführt. Der Vorteil dabei ist u.a. die einfache Verfahrensführung, der variable Einbau von Packungen oder Böden und damit die Anpassung an verschiedene Randbedingungen des Prozesses, z.B. Durchflüsse, Trennstufen etc.. Es handelt sich dabei vorzugsweise um gebräuchliche Packungskolonnen, Füllkörperkolonnen oder Bodenkolonnen wie Glockenboden- bzw. Siebbodenkolonnen.

Das erfindungsgemäße Verfahren erlaubt eine Verwendung von im Vergleich zu den bekannten Verfahren sehr geringen Lösemittelmengen, welche integriert in eine vorhandene Acroleinanlage ohne großen zusätzlichen apparativen oder Energieaufwand wiedergewonnen und wiederverwendet werden mit den weiteren bereits genannten positiven Wirkungen auf Durchsatz und Anlagenverfügbarkeit. Dies ist bei einem großtechnischen Prozess sowohl unter ökonomischer als auch ökologischer Hinsicht von großem Vorteil.

### Beschreibung des Ausführungsbeispiels

Zur näheren Erläuterung der Erfindung anhand des Ausführungsbeispiels wird auf Fig. 1 Bezug genommen, die eine Ausführungsform des erfindungsgemäßen Verfahrens zeigt.

Dem Stand der Technik entsprechend wird zunächst Propen verdampft und das gasförmige Propen (3) mit Luft (1), Dampf (2) und Kreisgas (19, nicht kondensierbares Prozessgas = rezirkuliertes Prozessabgas) gemischt und die Mischung dem Rohrbündelreaktor (entsprechend Stufe A) zugeführt. Dort erfolgt dann an geeigneten Katalysatoren die Umsetzung des Propens zu Acrolein und weiteren Nebenprodukten (Reaktion).

Das den Reaktor unten verlassende Prozessgas (4, Reaktionsgas) wird zunächst in einer ersten Kolonne (mit Böden und Packungen, im Weiteren als Quenchkolonne bezeichnet, entsprechend Stufe B) von Hochsiedern befreit. Zuerst erfolgt dazu im unteren Teil der Kolonne eine Kühlung des vorzugsweise mit 220- 250 °C eintretenden Prozessgases mittels eines Kühlkreislaufes. Im Mittelteil der Kolonne erfolgt dann die weitgehende Abreicherung der Hochsieder, insbesondere von Acrylsäure. In einem Kühlkreislauf im oberen Teil der Quenchkolonne erfolgt eine weitere Abkühlung des Prozessgases, das aus dem Mittelteil der Kolonne nach oben kommt, wobei aus dem gesättigten Gasstrom Füssigkeit auskondensiert wird, die als Rücklauf auf den Mittelteil der Kolonne zurückgeführt wird.

Dem Stand der Technik entsprechend wird nun das Acrolein aus dem Prozessgas, das aus der Quenchkolonne am Kopf austritt, in einer nachfolgenden Kolonne (mit strukturierten Packungen, im Weiteren als Absorptionskolonne bezeichnet, entsprechend Stufe C) absorbiert. Dazu wird dieses Prozessgas der Absorptionskolonne im Sumpfbereich zugeführt und vom Kolonnenkopf her mit Sorptionslösung gewaschen, wobei sich die Sorptionslösung mit Acrolein belädt. Das von Acrolein weitgehend befreit Prozessgas verlässt als Abgas (12) die Anlage und wird teilweise als Kreisgas (19, Verdünnungsgas, rezirkuliertes Prozessabgas) zur Inertisierung der Feedmischung des Reaktors zurückgeführt.

Weiterhin dem Stand der Technik entsprechend wird die mit Acrolein beladene Sorptionsflüssigkeit aus dem Sumpf der Absorptionskolonne (entsprechend Stufe C) abgezogen und etwa mittig einer Destillationskolonne (Bodenkolonne, auch als Desorptionskolonne bezeichnet, entsprechend Stufe D) zugeführt. Dort erfolgt die destillative Abtrennung des Acrolein aus der Sorptionslösung. Im Abtriebsteil der Kolonne wird die Sorptionslösung weitgehend von Acrolein befreit und kann dann auf die Absorptionskolonne zurückgeführt werden (10), wobei der Sorptionslösung noch in geringen Umfang VE Wasser (11) zugefügt wird. Im Verstärkungsteil der Kolonne wird das Acrolein bis nahe an den azeotropen Punkt aufkonzentriert und der austretende Acroleinbrüdenstrom in einem Einspritzkondensator (23) durch im Kreislauf zirkuliertes unterkühltes Acrolein auskondensiert, dem Stabilisator (13) zugesetzt wird. Als Stabilisator wird in der Regel Hydrochinon oder auch andere geeignete Stabilisatoren verwendet. Der für die Trennung notwendige Rücklauf wird auf die Kolonne zurückgeführt und das gewonnene Acrolein dem Acroleintanklager zugeführt (14).

Dieser bisher in wesentlichen Punkten dem Stand der Technik entsprechende Aufarbeitungsprozess wird nun erfindungsgemäß um die beiden Phasentrenner I und II im Kopfkreislauf der Quenchkolonne und in der Rückführung der Sorptionslösung (10) auf die Absorptionskolonne ergänzt und die Betriebsweise des Aufarbeitungsprozesses entsprechend der weiteren Beschreibung erfindungsgemaß geändert.

Dem üblicherweise als Sorptionsflüssigkeit verwendeten Wasser wird ein organisches Lösungsmittel zugesetzt, das kaum in Wasser löslich ist, eine organische Phase bei der Mischung mit Wasser ausbildet, und das vorzugsweise einen Siedepunkt aufweist von 150 -230°C, vorzugsweise von 170 -190 °C. Weiterhin bevorzugt sollte das Lösungsmittel auch eine gute Aufnahmefähigkeit für gebildete Oligomere und weitere organische Nebenprodukte aufweisen. Als besonders gut geeignet für diesen Zweck hat sich 2-Ethylhexanol erwiesen bzw. die damit gebildeten organischen Phasen. Zur Ergänzung von organischem Lösungsmittel wird dieses beispielsgemäß im unteren Bereich der Absorptionsstufe C eingetragen (5).

Um dem weiteren Zweck des Lösungsmittels, nämlich der Abtrennung von potentiell Polymer bildenden Verunreinigungen Rechnung zu tragen, muss die Lösungsmittelmatrix gereinigt werden. Dazu wird aus dem Teilstrom 6 der zur Absorptionskolonne (Schritt C) zurückgeführten Sorptionslösung 6 mit dem Phasentrenner II eine organische Phase II (LM II) abgetrennt. Ein Betrieb in einem Bereich von 10°C - 90 °C wird dabei eingehalten, in besonders geeigneter Weise ein Temperaturbereich von 15°C - 60 °C. Dementsprechend ist der Phasentrenner II nach dem als WT1 gekennzeichneten Wärmetauscher in den Prozess eingebunden. Im WT1 wird zur Optimierung des Energieverbundes die warme Sorptionsflüssigkeit aus dem Sumpf der Desorptionskolonne mit kalter beladener Sorptionsflüssigkeit aus dem Sumpf der Absorptionskolonne gekühlt und die beladene Sorptionsflüssigkeit vor Eintritt in die Desorptionskolonne vorgewärmt.

Die Lösungsmittel enthaltende organische Phase kann aus einem Teilstrom der Sorptionsflüssigkeit abgetrennt werden, es kann aber auch die rezirkulierte Sorptionsflüssigkeit insgesamt durch den Phasentrenner II geleitet werden. Aus dem Volumen des vollständig gefluteten Phasentrenners II und dem durchgeleiteten Volumenstrom an Flüssigkeit ergibt sich die Verweilzeit (VWZ) im Phasentrenner II. Eine hinreichend gute Trennung wird bei Verweilzeiten von 0,5 - 20 min erzielt, besonders bevorzugt einer VWZ von 1 -10 min und diese entsprechend eingestellt.

Die abgeschiedene organische Phase II (LM II) wird nun auf den oberen Boden des Sumpfkreislaufs in der Quenchkolonne (Stufe B) geleitet (7). Die aus dem Phasentrenner II abgezogene Menge wird dabei so bemessen, dass etwas mehr Flüssigkeit abgezogen wird, als LM II im Phasentrenner II anfällt und dadurch die Ausbildung einer Grenzschicht im Phasentrenner II vermieden wird. Dies hat sich als vorteilhaft erwiesen, da an der Grenzschicht vermehrt Polymere gebildet werden.

Aus der dem Sumpfkreislauf des Quenchers zugeführten organischen Phase II (7, LM II) werden dann durch das heiße Prozessgas die flüchtigen Lösungsmittelmatrixanteile im Gegensatz zu den sonstigen Hochsiedern abgestrippt, da die LM II eine zweite Phase ausbildet (Prinzip der Wasserdampfdestillation). Im oberen (den Kopfteil einbeziehenden) Kühlkreislauf kondensiert bei den tieferen Temperaturen die LM II zum großen Teil mit dem übrigen Kondensat aus und wird dann über einen im oberen Kühlkreislauf (8) befindlichen Phasentrenner I aus dem Kühlkreislauf abgetrennt. Die abgetrennte organische Phase I (LM I) wird in den Sumpf der Absorptionskolonne (Stufe C) zurückgeführt (9). Die Betriebsparameter für den Phasentrenner I gleichen in Bezug auf die VWZ denen für Phasentrenner II, die Arbeitstemperatur kann jedoch tiefer eingestellt werden, da die abdestillierte LM I eine niedrigere Viskosität aufweist, als die mit Hochsiedern beladene LM II aus Phasentrenner II.

Diese Betriebsweise erlaubt einen in weiten Bereichen frei einstellbaren Lösungsmittelstrom im dargestellten geschlossenen Kreislauf durch den warmen Teil der Desorptionskolonne (Stufe D), ohne dass größere Lösungsmittelverluste auftreten. Die einstellbare Menge ist vor allem durch die Destillationskapazität in der Quenchkolonne limitiert. Der Gehalt an LM stellt sich entsprechend der zugeführten Frischlösungsmittelmenge und den Verlusten an LM mit dem Abwasser (15, 16) und dem Abgas (18) ein. Der LM-Gehalt sollte mindestens so hoch sein, dass sich eine organische Phase (umfassend LM) ausbildet, zu hohe LM-Konzentrationen in der Sorptionslösung verschlechtern ggf. die Trennwirkung in der Absorptionskolonne. Beispielsgemäß vorteilhaft beträgtder LM- Gehalt in der Sorptionslösung 0,1 bis 5 Gew.% , bevorzugt 0,2-3 Gew.%.

Die durch das Lösungsmittel aufgenommenen Verunreinigungen verbleiben im Sumpf der Quenchkolonne und werden zusammen mit dem Abwasser verbrannt. Von Vorteil ist dabei, dass im acrylsäurehaltigen Abwasser (16) die Verunreinigungen gut genug löslich sind und keine separate organische Phase entsteht, welche den anschließenden Verbrennungsprozess durch schwankenden Heizwert beeinträchtigen könnte. Ein weiterer deutlicher Vorteil dieses Prozesses ist, dass die verfahrensbedingten Apparate genutzt werden und keine zusätzlichen Apparate für eine externe Lösungsmittelaufarbeitung notwendig werden. Der Lösungsmittelverbrauch ist soweit herabgesetzt, dass nur vergleichsweise geringe Lösungsmittelverluste im Abgas und im Abwasser ergänzt werden müssen.

Ein weiterer Vorteil dieser Prozessführung ist, dass der Lösungsmittelgehalt vor Aufgabe der Sorptionsflüssigkeit auf die Absorptionskolonne und vor Aufgabe des oberen Kühlkreislaufs auf die Quenchkolonne bzw. der Rücklauf der Quenchkolonne soweit im Lösungsmittelgehalt erniedrigt ist, dass an diesen Stellen in den Kolonnen Packungen (wie im Ausführungsbeispiel) eingesetzt werden können. Dies erlaubt bei gegebener Apparategröße bekanntermaßen einen höheren Durchsatz als bei der Nutzung von Böden als Kolonneneinbauten.

### Versuchsbeschreibung der Vergleichsbeispiele 1 bis 3

Die Vergleichsbeispiele wurden analog dem oben beschriebenem Beispiel ausgeführt jedoch mit den folgenden Unterschieden:

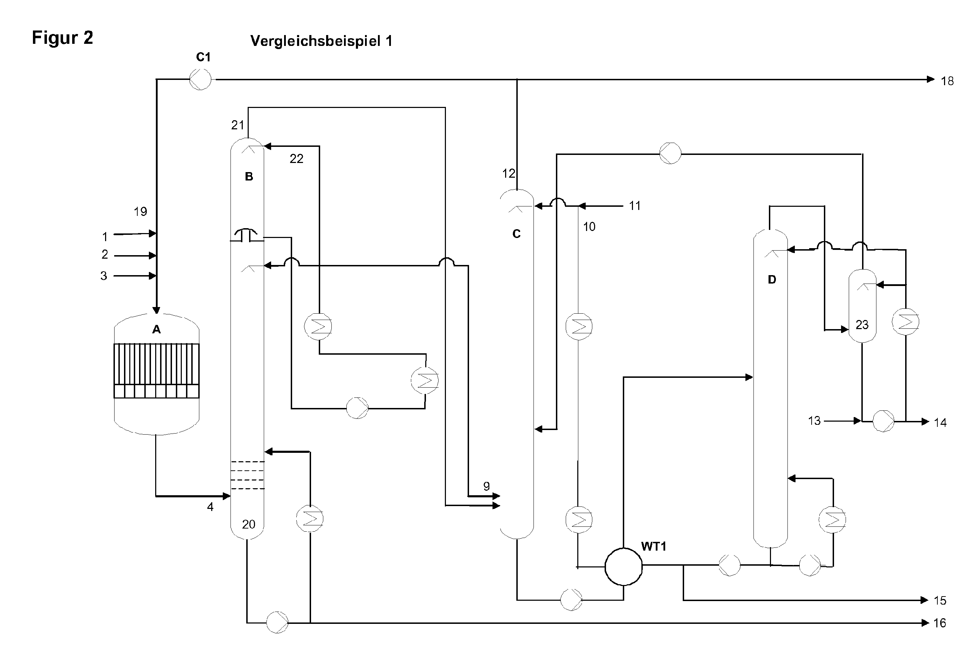

- Figur 2:: Im Gegensatz zu Verfahren gemäß Beispiel 1 (Figur 1) sind im Vergleichsbeispiel 1 der Phasentrenner I und Phasentrenner II nicht installiert und es wird dem Verfahren kein Lösungsmittel zugeführt.

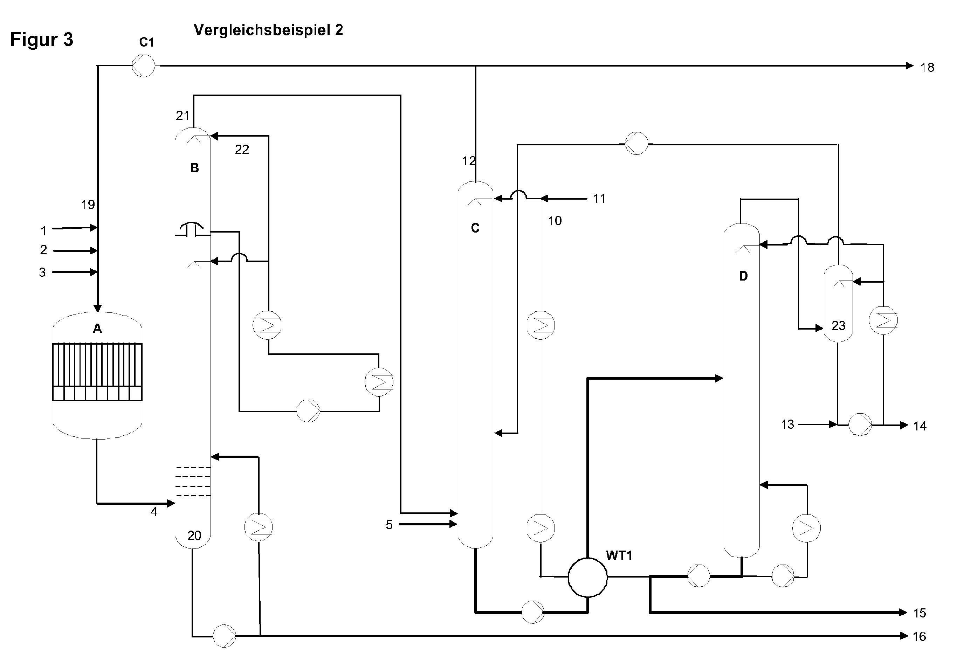

- Figur 3:: Im Gegensatz zu Verfahren gemäß Beispiel 1 (Figur 1) sind im Vergleichsbeispiel 2 der Phasentrenner I und Phasentrenner II nicht installiert und es wird dem Verfahren Lösungsmittel in den Sumpf der Absorption zugeführt. Dieses reichert sich in der Sorptionsflüssigkeit in gewissen Umfang an und wird zusammen mit dem Prozessabwasser aus dem Sorptionskreislauf (Strom 15) als verschmutzte org. Phase aus dem Prozess entfernt und thermisch entsorgt.

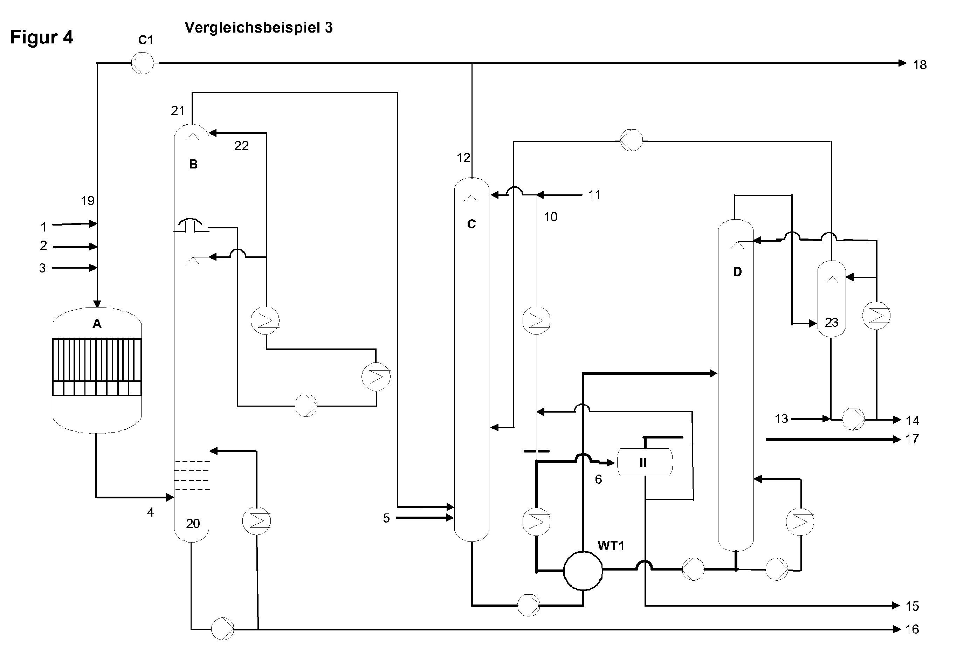

- Figur 4:: Im Gegensatz zu Verfahren gemäß Beispiel 1 (Figur 1) ist im Vergleichsbeispiel 3 der Phasentrenner II installiert und es wird dem Verfahren Lösungsmittel in den Sumpf der Absorption zugeführt. Die aus dem Phasentrenner II, der wie in Beispiel 1 betrieben wird, abgezogene organische Phase II (LM II) wird einer 2-stufigen Vakuumdestillation zugeführt. In einer ersten Stufe werden Leichtsieder abgetrennt, in einer zweiten Stufe der destillierbare Anteil der LM von Hochsiedern abdestilliert. Dieser Anteil wird zusammen mit dem frischen Lösungsmittel (Strom 5) wieder in den Sumpf der Absorptionsstufe zurückgeführt.

Die in den Beispielen (Vergleichsbeispielen) verwendeten Verfahrenseinstellungen sind in Tabelle 1 zusammengefasst.

**Tabelle 1: Verfahrenseinstellungen der Beispiele / Vergleichsbeispiele**

| **Nr. in den Fig.** | **Beschreibung** | **Einheit** | **Beispiel gemäß** **Figur 1** | **Vergleichsbeispiel 1 gemäß** **Figur 2** | **Vergleichsbeispiel 2 gemäß** **Figur 3** | **Vergleichsbeispiel 3 gemäß** **Figur 4** |
|---|---|---|---|---|---|---|
| 1 | Luft (trocken) | Nm3/h | 28500 | 24000 | 28500 | 28500 |
| 2 | Dampf | kg/h | 1500 | 1263 | 1500 | 1500 |
| 3 | Propen (Pe) | Nm3/h | 3800 | 3200 | 3800 | 3800 |
| 4 | Prozessgas | °C | 240 | 240 | 240 | 240 |
| 5 | 2 Ethylhexanol | kg/h | 14 | | 30 | 30 |
| | | kg/100kgPe | 0,2 | | 0,4 | 0,4 |
| | Destillat aus ext. Aufarbeitung | kg/h | n.a | n.a. | | 70 |
| 6 | Sorptionslösung | m3/h | 67,5 | n.a. | n.a. | 67,5 |
| | Temperatur | °C | 25 | n.a. | n.a. | 25 |
| | VWZ | Min | 6 | n.a. | n.a. | 6 |
| | Gehalt organischer Phase | Gew.% | 0,3 | n.a. | n.a. | 0,5 |
| 7 | organische Phase aus Phasentrenner II | kg/h | 60 | n.a. | n.a. | n.a. |
| | | kg/100kgPe | 0,8 | | | |
| 8 | Kühlkreislauf | m3/h | 60 | n.a. | n.a. | n.a. |
| | Temp. | °C | 20 | n.a. | n.a. | n.a. |
| | VWZ | Min. | 6 | n.a. | n.a. | n.a. |
| 9 | organische Phase aus Phasentrenner I | kg/h | 40 | n.a. | n.a. | n.a. |
| | | kg/100kgPe | 0,6 | | | |
| 10 | Sorptionslösung | m3/h | 135 | 120 | 135 | 135 |
| | | °C | 8 | 8 | 8 | 8 |
| 11 | VE Wasser | m3/h | 1,2 | 1,2 | 1,2 | 1,2 |
| 12 | Druck am Austritt aus der Absorptionsstufe | bara | 1,3 | 1,3 | 1,3 | 1,3 |
| 13 | Stabilisator (10 % Hydrochinon in Acrolein) | kg/h | 50 | 42 | 50 | 50 |
| 14 | Acrolein | kg/h | 8000 | 6737 | 8000 | 8000 |
| 15 | Abwasser aus dem Sorbatkreislauf | t/h | 1,2 | 1,2 | 1,2 | 1,2 |
| 16 | Abwasser aus der Quenchstufe | t/h | 5,4 | 4,6 | 5,4 | 5,4 |
| 17 | organische Phase aus Phasentrenner II zur externen Aufarbeitung | kg/h | n.a. | n.a. | n.a. | 100 |
| 18 | Prozessabgas zur Verbrennung | Nm3/h | 25600 | 21600 | 25600 | 25600 |
| 19 | Rezirkuliertes Prozessabgas | Nm3/h | 22000 | 18560 | 22000 | 22000 |
| 20 | Temperatur Sumpf Quencher | °C | 70 | 70 | 70 | 70 |
| 21 | Druck am Kopf des Quenchers | bara | 1,5 | 1,5 | 1,5 | 1,5 |
| 22 | Temperatur im Kopfkreislauf Quencher | °C | 14 | 14 | 14 | 14 |

| | | | | | | |
|---|---|---|---|---|---|---|
| n. a.: nicht anwendbar | | | | | | |

### Ergebnisse der Beispiele/Vergleichsbeispiele:

Die aufgeführten Beispiele/Vergleichsbeispiele wurden in jeweils einer Anlage durchgeführt, wie sie den in Figuren 1 bis 4 gezeigten Anlagen entspricht. Die angegebenen Ergebnisse sind dabei im Wesentlichen durch die beschriebenen Parameter bestimmt.
- Fig. 1: Erfindungsgemäßer Prozess Anlagenbetrieb > 1 Jahr bis Reinigung erforderlich wird Spez. Lösungsmittel-Verbrauch ca. 0,2 Gew.% Frischlösungsmittel bezogen auf die eingesetzte Propen-Menge Vergleichsbeispiele 1-3:
- Fig. 2: Es zeigt sich dabei, dass die maximal einstellbare Anlagenlast (d.h. Pe Einspeisung) auf etwa 80% der Anlagenlast aus Beispiel 1 begrenzt ist. Bei höherer Last wird das Betriebsverhalten der Anlage durch Schaumbildung in der Destillation instabil. Weiterhin wird beobachtet, dass selbst bei dieser niedrigeren Last bereits nach max. 3 Monaten eine Reinigung des Sumpfbereichs der Destillation erforderlich wird (Bildung von Polymerablagerungen). Kein Lösungsmittelverbrauch.
- Fig. 3: Es zeigt sich, dass in diesem Fall keine Produktionsminderung wegen Schaumbildung in der Destillation mehr notwendig wird und das Reinigungsintervall der Produktionsanlage auf bis zu 6 Monate verlängert wird. Der Frischlösungsmittelverbrauch liegt dabei bei ≥ 0,4 Gew.% Lösungsmittel bezogen auf die eingesetzte Propenmenge
- Fig. 4: Es zeigt sich dabei, dass ein Anlagenbetrieb wie in Beispiel 1 ermöglicht wird, der Frischlösungsmittelverbrauch dabei aber auch bei ≥ 0,4 Gew.% Lösungsmittel bezogen auf die eingesetzte Propenmenge liegt. Das Investitionsvolumen für die Vakuumdestillationsanlage ist wesentlich höher als für den Phasentrenner I und der Betrieb aufgrund der teerartigen Konsistenz des Destillationsrückstandes störanfällig

**Bezugszeichentabelle**

| **Nr. in Figuren** | **Beschreibung** |
|---|---|
| 1 | Luft (trocken) |
| 2 | Dampf |
| 3 | Propen |
| 4 | Prozessgas |
| 5 | 2 Ethylhexanol Destillat aus ext. Aufarbeitung |
| 6 | Sorptionslösung |
| 7 | organische Phase aus Phasentrenner II |
| 8 | Kühlkreislauf |
| 9 | organische Phase aus Phasentrenner I |
| 10 | Sorptionslösung |
| 11 | VE Wasser |
| 12 | Druck am Austritt aus der Absorptionsstufe |
| 13 | Stabilisator (10 % Hydrochinon in Acrolein) |
| 14 | Acrolein |
| 15 | Abwasser aus dem Sorbatkreislauf |
| 16 | Abwasser aus der Quenchstufe |
| 17 | organische Phase aus Phasentrenner II zur externen Aufarbeitung |
| 18 | Prozessabgas zur Verbrennung |
| 19 | Rezirkuliertes Prozessabgas |
| 20 | Temperatur Sumpf Quencher |
| 21 | Druck am Kopf des Quenchers |
| 22 | Temperatur im Kopfkreislauf Quencher |
| 23 | Einspritzkondensator |
| WT1 | Wärmetauscher 1 |

## Patentansprüche

1. Kontinuierliches Verfahren zur Herstellung von Acrolein, bei dem die nachfolgenden Schritte durchgeführt werden
A) Gasphasenoxidation von Propen mit Hilfe von Luft an einem heterogenen Katalysator in Anwesenheit eines Verdünnungsgases in einer Reaktionsstufe
B) Auffangen des Acrolein-haltigen Gasstromes aus A) in einer Quenchstufe bestehend aus Sumpf-, Mittel- und Kopfteil zur Abtrennung von Nebenprodukten in Gegenwart von Wasser und einer kaum wasserlösliches organisches Lösungmittel enthaltenden organischen Phase
C) Auffangen des Acrolein-haltigen Gasstromes aus der Quenchstufe B) in einer Absorptionsstufe bestehend aus Sumpf-, Mittel- und Kopfteil in Gegenwart von Wasser und einer kaum wasserlösliches organisches Lösungmittel enthaltenden organischen Phase zur Gewinnung einer organische Phase enthaltenden wässrigen Acrolein-Lösung und eines nicht kondensierbaren Gasstromes
C1) zumindest teilweise Rückführung des nicht kondensierbaren Gasstromes aus C) als Verdünnungsgas in die Reaktionsstufe A)
D) Destillative Abtrennung des Acroleins aus der organische Phase enthaltenden wässrigen Acrolein-Lösung aus C) in einer Destillationsstufe
wobei die verbleibende vom Acrolein befreite, organische Phase enthaltende wässrige Lösung aus der Destillationsstufe D) ausgetragen wird, so dass sich eine organische Phase II bildet, die vom zugehörigen wässrigen Anteil II getrennt wird (Phasentrennung II), die organische Phase II in den Sumpfteil der Quenchstufe B) eingetragen wird, aus welchem die organische Phase II durch Destillation und /oder Strippung zusammen mit einem wässrigen Anteil über den Kopfteil abgetrennt wird, so dass sich eine organische Phase I bildet, welche vom zugehörigen wässrigen Anteil I getrennt wird (Phasentrennung I) und in den Sumpfteil der Absorptionsstufe C) eingetragen wird und wobei das kaum wasserlösliche organische Lösungsmittel eine Wasserlöslichkeit von ≤ 20g/l bei 20°C aufweiset.

2. Verfahren gemäß Anspruch 1, **dadurch gekennzeichnet, dass** permanent oder intermittierend kaum wasserlösliches organisches Lösungsmittel zur Ergänzung der organischen Phase an geeigneter Stelle des Verfahrens eingebracht wird.

3. Verfahren gemäß Anspruch 2, **dadurch gekennzeichnet, dass** das organische Lösungsmittel in den Sumpfteil der Absorptionsstufe und/oder der Destillationsstufe eingebracht wird.

4. Verfahren gemäß Anspruch 2 oder 3, **dadurch gekennzeichnet, dass** ein organisches Lösungsmittel eingesetzt wird, das einen Siedepunkt bei Normaldruck von 150 bis 230 °C, vorzugsweise von 170 bis 190 °C aufweist.

5. Verfahren gemäß Anspruch 4, **dadurch gekennzeichnet, dass** als organisches Lösungsmittel ein verzweigter bzw. unverzweigter offenkettiger C6- bis C10-Alkohol, vorzugsweise 2-Ethylhexanol verwendet wird.

6. Verfahren gemäß Anspruch 2 oder 3, **dadurch gekennzeichnet, dass** bezogen auf die in die Reaktionsstufe A) eingetragenen Menge an Propen 0,05 bis 1 Gew.%, vorzugsweise zwischen 0,1 und 0,5 Gew.% organisches Lösungsmittel ergänzt wird, vorzugsweise soviel organisches Lösungsmittel ergänzt wird, dass ein gleichbleibender Gehalt an organischer Phase aufrechterhalten wird.

7. Verfahren gemäß einem der Ansprüche 1 bis 6, **dadurch gekennzeichnet, dass** der Acrolein-haltige Gasstrom aus A) mit einer Temperatur von 200-280 °C vorzugsweise von 220-250 °C in den Sumpfteil des entsprechenden Schritts B) gelangt.

8. Verfahren gemäß einem der Ansprüche 1 bis 7, **dadurch gekennzeichnet, dass** in der Quenchstufe B die Sumpftemperatur zwischen 60 und 90 °C bei einem Druck im Kopfteil von 1,2 bis 1,8 bar so eingestellt wird, dass keine separate organische Phase im Sumpfteil der Quenchstufe B vorhanden ist.

9. Verfahren gemäß Anspruch 8 , **dadurch gekennzeichnet, dass** die Sumpftemperatur der Quenchstufe B eingestellt wird, indem ein Teilstrom der dort kondensierten Flüssigkeit abgeführt wird und nach entsprechendem Abkühlen dem unteren Drittel der Quenchstufe B wieder zugeführt wird (unteres Umpumpen).

10. Verfahren gemäß Anspruch 9, **dadurch gekennzeichnet, dass** aus dem abgeführten Teilstrom der kondensierten Flüssigkeit ein Teilstrom aus dem Verfahren ausgeschleust wird.

11. Verfahren gemäß einem der Ansprüche 1 bis 10, **dadurch gekennzeichnet, dass** ein Teilstrom der im Kopfteil der Quenchstufe B vorhandenen Kondensats abgeführt wird, vorzugsweise auf < 20°C gekühlt und dem Kopfteil der Quenchstufe B wieder zugeführt wird, wodurch der aus dem Mitteilteil der Quenchstufe B eintretende Acrolein-haltige Gasstrom gekühlt wird und fortwährend weiteres Kondensat erzeugt wird, welches als Rücklauf in den Mittelteil der Quenchstufe zurückgeführt wird (oberes Umpumpen).

12. Verfahren gemäß Anspruch 11, **dadurch gekennzeichnet, dass** ein Teilstrom des Kondensats der Phasentrennung I zugeführt wird.

13. Verfahren gemäß einem der Ansprüche 1 bis12, **dadurch gekennzeichnet, dass** die Phasentrennung I bei einer Temperatur von 8 bis 60 °C, vorzugsweise von 10 bis 50 °C besonders bevorzugt von 12 bis 40°C durchgeführt wird.

14. Verfahren gemäß einem der Ansprüche 1 bis13, **dadurch gekennzeichnet, dass** die Phasentrennung I mit einer Verweilzeit von 0,5 bis 20 min, vorzugsweise von 1 bis 10 min durchgeführt wird.

15. Verfahren gemäß einem der Ansprüche 11 bis 14, **dadurch gekennzeichnet, dass** der wässrige Anteil I aus der Phasentrennung I zumindest teilweise dem Rücklauf des erzeugten Kondensats zugeführt wird.

16. Verfahren gemäß mindestens einer der Ansprüche 1 bis 15, **dadurch gekennzeichnet, dass** die aus der Destillationsstufe D ausgetragene vom Acrolein befreite, organische Phase enthaltende wässrige Lösung (Sorptionslösung) teilweise direkt auf den Kopfteil der Absorptionsstufe C zurückgeführt wird und der restliche Teil der Phasentrennung II zugeführt wird.

17. Verfahren gemäß einem der Ansprüche 1 bis 16, **dadurch gekennzeichnet, dass** die Phasentrennung II bei einer Temperatur von 10 bis 90 °C, vorzugsweise von 15 bis 60 °C, besonders bevorzugt von 20 bis 40 °C durchgeführt wird.

18. Verfahren gemäß einem der Ansprüche 1 bis 17, **dadurch gekennzeichnet, dass** die Phasentrennung II mit einer Verweilzeit von 0,5 bis 20 min, vorzugsweise von 1 bis 10 min durchgeführt wird.

19. Verfahren gemäß einem der Ansprüche 1 bis 18, **dadurch gekennzeichnet, dass** die Menge an organischer Phase II, die in die Quenchstufe B) eingetragen wird 0,1 bis 3 Gew.%, vorzugsweise 0,2 bis 1 Gew.%, bezogen auf die insgesamt in die Reaktionsstufe A) eingetragenen Mengen an Propen beträgt.

20. Verfahren gemäß einem der Ansprüche 1 bis 19, **dadurch gekennzeichnet, dass** der wässrige Anteil II aus der Phasentrennung II teilweise zur Absorptionsstufe C), vorzugsweise zum Kopfteil der Absorptionsstufe, zurückgeführt wird und vorzugsweise ein weiterer Teilstrom des wässrigen Anteils II aus dem Verfahren ausgeschleust wird.

21. Verfahren gemäß einem der Ansprüche 1 bis 20, **dadurch gekennzeichnet, dass** der Gehalt an organischer Phase in der Sorptionslösung 0,1 bis 5 Gew.% beträgt, vorzugsweise 0,2 bis 3 Gew.%.

22. Verfahren gemäß einem der Ansprüche 16 oder 21 **dadurch gekennzeichnet, dass** die Sorptionslösung mit einer Temperatur von 5 - 18°C, vorzugsweise 6 - 12°C und bei einem Druck am Kopfteil der Absorptionsstufe C) zwischen 1,1 und 1,7 bar in die Absorptionsstufe C) eingebracht wird.

23. Verfahren gemäß mindestens einer der Ansprüche 1 bis 22, **dadurch gekennzeichnet, dass** die Quench- , Absorptions- und/oder Destillationsstufe jeweils in Form einer Kolonne ausgeführt ist.

## Claims

1. Continuous process for preparing acrolein, wherein the following steps are carried out
A) gas-phase oxidation of propene by means of air over a heterogeneous catalyst in the presence of a diluent gas in one reaction stage
B) capture of the acrolein-containing gas stream A) in a quenching stage consisting of bottom, middle and top sections for separating off by-products in the presence of water and an organic phase containing sparingly water-soluble organic solvent
C) capture of the acrolein-containing gas stream from the quenching stage B) in an absorption stage consisting of bottom, middle and top sections in the presence of water and an organic phase containing sparingly water-soluble organic solvent to obtain an aqueous acrolein solution which contains organic phase, and an incondensable gas stream
C1) at least partial recirculation of the incondensable gas stream from C) as diluent gas to the reaction stage A)
D) separation of the acrolein by distillation from the aqueous acrolein solution which contains organic phase from C) in a distillation stage
where the remaining aqueous solution which contains organic phase and has been freed of acrolein is discharged from the distillation stage D) so as to form an organic phase II which is separated from the associated aqueous fraction II (phase separation II), the organic phase II is introduced into the bottom section of the quenching stage B) from which the organic phase II is separated off together with an aqueous fraction via the top section by distillation and/or stripping so as to form an organic phase I which is separated from the associated aqueous fraction I (phase separation I) and introduced into the bottom section of the absorption stage C), and where the sparingly water-soluble organic solvent has a solubility in water of ≤ 20g at 20°C.

2. Process according to Claim 1, **characterized in that** organic solvent which is sparingly water-soluble is introduced continuously or intermittently to supplement the organic phase at a suitable point in the process.

3. Process according to Claim 2, **characterized in that** the organic solvent is introduced into the bottom section of the absorption stage and/or of the distillation stage.

4. Process according to Claim 2 or 3, **characterized in that** an organic solvent which has a boiling point at atmospheric pressure of from 150 to 230°C, preferably from 170 to 190°C, is used.

5. Process according to Claim 4, **characterized in that** a branched or unbranched open-chain C6- to C10-alcohol, preferably 2-ethylhexanol, is used as organic solvent.

6. Process according to Claim 2 or 3, **characterized in that** in a further amount of from 0.05 to 1% by weight, preferably from 0.1 to 0.5% by weight, of organic solvent, based on the amount of propene introduced into the reaction step A), is added, preference being given to addition of such an amount of organic solvent that a constant content of organic phase is maintained.

7. Process according to any of Claims 1 to 6, **characterized in that** the acrolein-containing gas stream from A) goes at a temperature of 200-280°C, preferably 220-250°C, into the bottom section of the corresponding step B).

8. Process according to any of Claims 1 to 7, **characterized in that** the temperature at the bottom of the quenching stage B is set in the range from 60 to 90°C at a pressure in the top section of from 1.2 to 1.8 bar so that no separate organic phase is present in the bottom section of the quenching stage B.

9. Process according to Claim 8, **characterized in that** the temperature at the bottom of the quenching stage B is set by discharging a substream of the liquid condensed there and, after appropriate cooling, feeding it back into the lower third of the quenching stage B (lower pump circulation).

10. Process according to Claim 9, **characterized in that** a substream of the discharged substream of the condensed liquid is removed from the process.

11. Process according to any of Claims 1 to 10, **characterized in that** a substream of the condensate present in the top section of the quenching stage B is discharged, preferably cooled to < 20°C and fed back into the top section of the quenching stage B, as a result of which the acrolein-containing gas stream coming in from the middle section of the quenching stage B is cooled and further condensate is continually produced and this is recirculated as runback to the middle section of the quenching stage (upper pump circulation).

12. Process according to Claim 11, **characterized in that** a substream of the condensate is fed to the phase separation I.

13. Process according to any of Claims 1 to 12, **characterized in that** the phase separation I is carried out at a temperature of from 8 to 60°C, preferably from 10 to 50°C, particularly preferably from 12 to 40°C.

14. Process according to any of Claims 1 to 13, **characterized in that** the phase separation I is carried out with a residence time of from 0.5 to 20 minutes, preferably from 1 to 10 minutes.

15. Process according to any of Claims 11 to 14, **characterized in that** the aqueous fraction I from the phase separation I is at least partly introduced into the runback of the condensate produced.

16. Process according to at least one of Claims 1 to 15, **characterized in that** the aqueous solution (sorption solution) which has been discharged from the distillation stage D and has been freed of acrolein and contains organic phase is partly recirculated directly to the top section of the absorption stage C and the remaining part is fed to the phase separation II.

17. Process according to any of Claims 1 to 16, **characterized in that** the phase separation II is carried out at a temperature of from 10 to 90°C, preferably from 15 to 60°C, particularly preferably from 20 to 40°C.

18. Process according to any of Claims 1 to 17, **characterized in that** the phase separation II is carried out with a residence time of from 0.5 to 20 minutes, preferably from 1 to 10 minutes.

19. Process according to any of Claims 1 to 18, **characterized in that** the amount of organic phase II introduced into the quenching stage B) is from 0.1 to 3% by weight, preferably from 0.2 to 1% by weight, based on the total amount of propene introduced into the reaction stage A).

20. Process according to any of Claims 1 to 19, **characterized in that** the aqueous fraction II from the phase separation II is partly recirculated to the absorption stage C), preferably to the top section of the absorption stage, and a further substream of the aqueous fraction II is preferably discharged from the process.

21. Process according to any of Claims 1 to 20, **characterized in that** the content of organic phase in the sorption solution is from 0.1 to 5% by weight, preferably from 0.2 to 3% by weight.

22. Process according to either Claim 16 or 21, **characterized in that** the sorption solution is introduced into the absorption stage C) at a temperature of 5 - 18°C, preferably 6 - 12°C, and at a pressure in the top section of the absorption stage C) in the range from 1.1 to 1.7 bar.

23. Process according to at least one of Claims 1 to 22, **characterized in that** the quenching, absorption and/or distillation stages are each configured in the form of a column.

## Revendications

1. Procédé continu de fabrication d'acroléine, selon lequel les étapes suivantes sont réalisées :
A) l'oxydation en phase gazeuse de propène à l'aide d'air sur un catalyseur hétérogène en présence d'un gaz diluant dans une étape de réaction,
B) le recueillement du courant gazeux contenant de l'acroléine de A) dans une étape de trempe constituée d'une partie de fond, intermédiaire et de tête pour la séparation de produits secondaires en présence d'eau et d'une phase organique contenant un solvant organique peu soluble dans l'eau,
C) le recueillement du courant gazeux contenant de l'acroléine de l'étape de trempe B) dans une étape d'absorption constituée d'une partie de fond, intermédiaire et de tête en présence d'eau et d'une phase organique contenant un solvant organique peu soluble dans l'eau pour l'obtention d'une solution d'acroléine aqueuse contenant une phase organique et d'un courant gazeux non condensable,
C1) le recyclage au moins partiel du courant gazeux non condensable de C) en tant que gaz diluant dans l'étape de réaction A),
D) la séparation par distillation de l'acroléine de la solution d'acroléine aqueuse contenant une phase organique de C) dans une étape de distillation,
la solution aqueuse restante, débarrassée de l'acroléine, contenant une phase organique, étant déchargée de l'étape de distillation D), de sorte qu'une phase organique II se forme, qui est séparée de la fraction aqueuse II associée (séparation de phases II), la phase organique II étant introduite dans la partie de fond de l'étape de trempe B), à partir de laquelle la phase organique II est séparée par distillation et/ou extraction conjointement avec une fraction aqueuse par la partie de tête, de sorte qu'une phase organique I se forme, qui est séparée de la fraction aqueuse I associée (séparation de phases I) et introduite dans la partie de fond de l'étape d'absorption C), le solvant organique peu soluble dans l'eau présentant une solubilité dans l'eau ≤ 20 g/l à 20 °C.

2. Procédé selon la revendication 1, **caractérisé en ce qu'**un solvant organique peu soluble dans l'eau est introduit de manière permanente ou intermittente à un emplacement approprié du procédé pour compléter la phase organique.

3. Procédé selon la revendication 2, **caractérisé en ce que** le solvant organique est introduit dans la partie de fond de l'étape d'absorption et/ou de l'étape de distillation.

4. Procédé selon la revendication 2 ou 3, **caractérisé en ce qu'**un solvant organique qui présente un point d'ébullition à pression normale de 150 à 230 °C, de préférence de 170 à 190 °C, est utilisé.

5. Procédé selon la revendication 4, **caractérisé en ce qu'**un alcool en C6 à C10 ramifié ou non ramifié à chaîne ouverte, de préférence le 2-éthylhexanol, est utilisé en tant que solvant organique.

6. Procédé selon la revendication 2 ou 3, **caractérisé en ce que**, par rapport à la quantité de propène introduite dans l'étape de réaction A), 0,05 à 1 % en poids, de préférence entre 0,1 et 0,5 % en poids, de solvant organique est ajouté, de préférence suffisamment de solvant organique pour maintenir une teneur constante en phase organique.

7. Procédé selon l'une quelconque des revendications 1 à 6, **caractérisé en ce que** le courant gazeux contenant de l'acroléine de A) atteint la partie de fond de l'étape B) correspondante à une température de 200 à 280 °C, de préférence de 220 à 250 °C.

8. Procédé selon l'une quelconque des revendications 1 à 7, **caractérisé en ce que**, dans l'étape de trempe B, la température de fond est ajustée entre 60 et 90 °C à une pression dans la partie de tête de 1,2 à 1,8 bar, de sorte qu'aucune phase organique séparée ne soit présente dans la partie de fond de l'étape de trempe B.

9. Procédé selon la revendication 8, **caractérisé en ce que** la température de fond de l'étape de trempe B est ajustée par déchargement d'un courant partiel du liquide qui s'y condense et réintroduction après un refroidissement approprié dans le tiers inférieur de l'étape de trempe B (circulation inférieure).

10. Procédé selon la revendication 9, **caractérisé en ce qu'**un courant partiel du courant partiel de liquide condensé déchargé est évacué du procédé.

11. Procédé selon l'une quelconque des revendications 1 à 10, **caractérisé en ce qu'**un courant partiel du condensat présent dans la partie de tête de l'étape de trempe B est déchargé, de préférence refroidi à < 20 °C et réintroduit dans la partie de tête de l'étape de trempe B, le courant gazeux contenant de l'acroléine entrant à partir de la partie intermédiaire de l'étape de trempe B étant ainsi refroidi et un condensat supplémentaire étant généré en continu, qui est recyclé en tant que reflux dans la partie intermédiaire de l'étape de trempe (circulation supérieure).

12. Procédé selon la revendication 11, **caractérisé en ce qu'**un courant partiel du condensat de la séparation de phases I est introduit.

13. Procédé selon l'une quelconque des revendications 1 à 12, **caractérisé en ce que** la séparation de phases I est réalisée à une température de 8 à 60 °C, de préférence de 10 à 50 °C, de manière particulièrement préférée de 12 à 40 °C.

14. Procédé selon l'une quelconque des revendications 1 à 13, **caractérisé en ce que** la séparation de phases I est réalisée avec un temps de séjour de 0,5 à 20 min, de préférence de 1 à 10 min.

15. Procédé selon l'une quelconque des revendications 11 à 14, **caractérisé en ce que** la fraction aqueuse I de la séparation de phases I est introduite au moins en partie dans le reflux du condensat généré.

16. Procédé selon au moins l'une quelconque des revendications 1 à 15, **caractérisé en ce que** la solution aqueuse contenant une phase organique, débarrassée de l'acroléine, déchargée de l'étape de distillation D (solution de sorption) est en partie recyclée directement dans la partie de tête de l'étape d'absorption C, et la partie restante est introduite dans la séparation de phases II.

17. Procédé selon l'une quelconque des revendications 1 à 16, **caractérisé en ce que** la séparation de phases II est réalisée à une température de 10 à 90 °C, de préférence de 15 à 60 °C, de manière particulièrement préférée de 20 à 40 °C.

18. Procédé selon l'une quelconque des revendications 1 à 17, **caractérisé en ce que** la séparation de phases II est réalisée avec un temps de séjour de 0,5 à 20 min, de préférence de 1 à 10 min.

19. Procédé selon l'une quelconque des revendications 1 à 18, **caractérisé en ce que** la quantité de phase organique II qui est introduite dans l'étape de trempe B) est de 0,1 à 3 % en poids, de préférence de 0,2 à 1 % en poids, par rapport aux quantités de propène introduites au total dans l'étape de réaction A).

20. Procédé selon l'une quelconque des revendications 1 à 19, **caractérisé en ce que** la fraction aqueuse II de la séparation de phases II est recyclée en partie dans l'étape d'absorption C), de préférence dans la partie de tête de l'étape d'absorption, et un autre courant partiel de la fraction aqueuse II est de préférence évacué du procédé.

21. Procédé selon l'une quelconque des revendications 1 à 20, **caractérisé en ce que** la teneur en phase organique dans la solution de sorption est de 0,1 à 5 % en poids, de préférence de 0,2 à 3 % en poids.

22. Procédé selon l'une quelconque des revendications 16 ou 21, **caractérisé en ce que** la solution de sorption est introduite à une température de 5 à 18 °C, de préférence de 6 à 12 °C, et à une pression dans la partie de tête de l'étape d'absorption C) comprise entre 1,1 et 1,7 bar, dans l'étape d'absorption C).

23. Procédé selon au moins l'une quelconque des revendications 1 à 22, **caractérisé en ce que** les étapes de trempe, d'absorption et/ou de distillation sont chacune configurées sous la forme d'une colonne.
